# EUROPÄISCHE PATENTSCHRIFT

(11) **EP 1 018 950 B1**
(45) Veröffentlichungstag und Bekanntmachung des Hinweises auf die Patenterteilung: **12.05.2004**
(21) Anmeldenummer: 98948776.4
(22) Anmeldetag: 07.08.1998
(51) Int. Cl.: A61B 18/08, A61B 17/22

(54) **VORRICHTUNG ZUR REVASKULARISATION VON MUSKELGEWEBE**
DEVICE FOR REVASCULARIZING MUSCULAR TISSUES
DISPOSITIF DE REVASCULARISATION DE TISSUS MUSCULAIRES

(30) Priorität: 17.09.1997 DE 19740825
(43) Veröffentlichungstag der Anmeldung: 19.07.2000
(73) Patentinhaber: Celon AG medical instruments, 14513 Teltow (DE)
(72) Erfinder: MÜLLER, Gerhard, D-14129 Berlin (DE); DESINGER, Kai, D-10827 Berlin (DE); SCHALDACH, Brita, D-13581 Berlin (DE)
(74) Vertreter: Eisenführ, Speiser & Partner
(86) Internationale Anmeldenummer: PCT/DE1998/002321
(87) Internationale Veröffentlichungsnummer: WO 1999/013784

(56) Entgegenhaltungen:
- EP-A- 0 688 536
- EP-A- 0 808 607
- WO-A-97/18768

## Beschreibung

Die Erfindung betrifft eine Vorrichtung zur Revaskularisation von Muskelgewebe durch Erzeugung röhrenförmiger Nekrosen.

Derartige Vorrichtungen sind z.B. aus WO-A-97 18768 bekannt und finden zunehmend klinische Anwendung, insbesondere auf dem Gebiet der Herzchirurgie.

Unter dem Schlagwort "Transmyokardiale Laser-Revaskularisierung" werden derzeit überwiegend drei unterschiedliche Lasersysteme, nämlich gepulste CO₂-Laser, gepulste Holmium-YAG-Laser und gepulste Excimer-Laser eingesetzt, und zwar vorrangig Geräte der US-Firmen PLC, CARDIO GENESIS und United States Surgical Corporation. Mit diesen gepulsten Lasersystemen gelingt unter Ausnutzung des Mechanismus der sogenannten Photoablation das Anlegen von transmyokardialen Kanälen, und systembedingt entstehen hierbei thermisch beeinflußte Randzonen und durch den Prozeß der Photoablation auch Stoßwellen. Diese Systeme sind extrem teuer, und dennoch sind Amplitude und Tiefenwirkung der Stoßwellen praktisch nicht optimierbar.

Der Erfindung liegt daher die Aufgabe zugrunde, ein vergleichsweise einfaches und kostengünstiges Gerät zur Revaskularisation von Muskelgewebe, speziell Herzmuskelgewebe, bereitzustellen, das verbesserte Möglichkeiten zur Optimierung der Behandlungsparameter bietet.

Die Aufgabe wird durch eine Vorrichtung mit den im Anspruchs 1 angegebenen Merkmalen gelöst.

Die Erfindung schließt die technische Lehre ein, eine Vorrichtung anzugeben, mit der unabhängig voneinander die Wandung einer im Zuge der Revaskularisation erzeugten röhrenförmigen Nekrose bzw. eines Kanals gezielt thermisch beeinflußt werden kann und mit der gleichzeitig in der Umgebung der Wandung stoßwellenartige Druckamplituden mit separat einstellbaren Parametern erzeugt werden können.

Sie schließt weiter den Gedanken ein, im Interesse einer getrennten Steuerung der Parameter verschiedene Energiequellen zur Erzeugung der thermischen Effekte einerseits und der Stoßwelleneffekte andererseits einzusetzen. Nach Untersuchungen der Erfinder ist der kombinierte Einsatz von HF-Energie und elektrischen Impulsen mit relativ hohen Feldstärken eines elektrischen Funkenstoßwellengenerators besonders kostengünstig und zeigt vorteilhafte Wirkungen. Die HF-Energie liegt zweckmäßigerweise im Bereich von einigen Watt und die Impuls-Feldstärke bei einigen kV/cm.

Überraschenderweise hat sich gezeigt, daß es bei Einsatz von bifilar, helixartig gewickelten Hochfrequenzelektroden, die isoliert auf einer Punktionsnadel angebracht werden, beim Einbringen der Hochfrequenzenergien und deren Verteilung in das randständige Gewebe zu einer Schrumpfung dieses Gewebes kommt. Nach Entfernen der Punktionsnadel bleibt ein offener Kanal zurück. Gleichzeitig kann durch gezielte Variation der Hochfrequenzenergie der Wärmeeintrag in die Kanalwand im Hinblick auf die Ausdehnung und die Konsistenz des verbleibenden Gewebes gezielt variiert werden. Ähnliche Effekte wurden auch mit anderen HF-Elektrodenkonfigurationen erzielt.

Durch zusätzliche Anbringung zweier weiterer voneinander getrennt isolierter Hochspannungselektroden, die am Ende oder entlang des HF-Applikationssytems (der HF-Punktionsnadel) freiliegen können, kann dann zudem durch kurzzeitiges Anlegen von potentialfreier Hochspannung ein funkenähnlicher Durchschlag erzeugt werden, der wiederum Stoßwellen in der Umgebung erzeugt.

Durch Variation der Hochspannung und der Dauer der Hochspannungsimpulse kann - unabhängig vom Eintrag von thermisch wirksamer HF-Energie über die bipolar angeordneten HF-Elektroden - die Stärke der Stoßwellenamplituden getrennt variiert werden. Durch Wahl verschiedener bipolarer Elektrodenkonfigurationen kann auf die thermische Koagulationszone Einfluß genommen werden.

In einem bevorzugten Ausführungsbeispiel besteht die HF-Punktionsnadel aus einer bruchfesten Keramik-Metall-Verbundkapillare, auf deren äußerer Wandung die bipolaren HF-Elektroden aufgebracht sind und in deren Innerem die Zuleitungen der HF-Elektroden isoliert voneinander geführt und am distalen Ende randständig fixiert sind. Als Elektrodenträger können aber auch andere geeignete Materialien, wie etwa hochtemperaturfeste Kunststoffe (PEEK, PPSU etc.) dienen.

Die HF-Punktionsnadel ist gemäß einem bevorzugten Ausführungsbeispiel in einer elektrisch, mechanisch oder hydraulisch betriebenen Vorschubeinrichtung, die sich im Inneren eines Handstückes befindet, auswechselbar angebracht. Das Handstück selbst ist in vorteilhafter Weise mit einer distalen Fixierhalterung dergestalt ausgebildet, daß beim Einsatz des Gerätes zur Perforation des Herzmuskels eine Verankerung im Epikard möglich ist und mittels der Vorschubeinrichtung die Punktionsnadel kontrolliert in den Herzmuskel eingebracht werden kann.

Die Energiezufuhr für die HF-Elektroden erfolgt durch einen bipolaren Hochfrequenzgenerator mit einstellbarer Leistung und entsprechend angepaßter Abschlußimpedanz, die Versorgung der Hochspannungsimpuls-Elektroden erfolgt durch einen Hochspannungsimpulsgenerator.

In Weiterführung des Erfindungsgedankens kann für die Erzeugung größerer Kanaldurchmesser anstelle der Punktionsnadel auch ein rotierendes Hohlmesser vorgesehen sein, durch das die Kontur des Kanals in das Gewebe eingeschnitten wird. Die Anbringung einer Absaugpumpe an das oben beschriebene Handstück ermöglicht es, das überständige Gewebe im Inneren des Hohlmessers abzusaugen.

Vorteilhafte Weiterbildungen der Erfindung sind im übrigen in den Unteransprüchen gekennzeichnet bzw. werden nachstehend im Rahmen der Beschreibung der bevorzugten Ausführung der Erfindung anhand der Figuren näher dargestellt. Es zeigen:
- Figur 1: eine Vorrichtung gemäß einem Ausführungsbeispiel der Erfindung in Längsschnittdarstellung,
- Figur 2a bis 2c: weitere Ausführungsformen in Seitenansichten
- Figur 3: eine Querschnittsdarstellung der Hohlspitze der Vorrichtung nach Fig. 1 und
- Figur 4: eine Seitenansicht einer weiteren Ausführungsform.

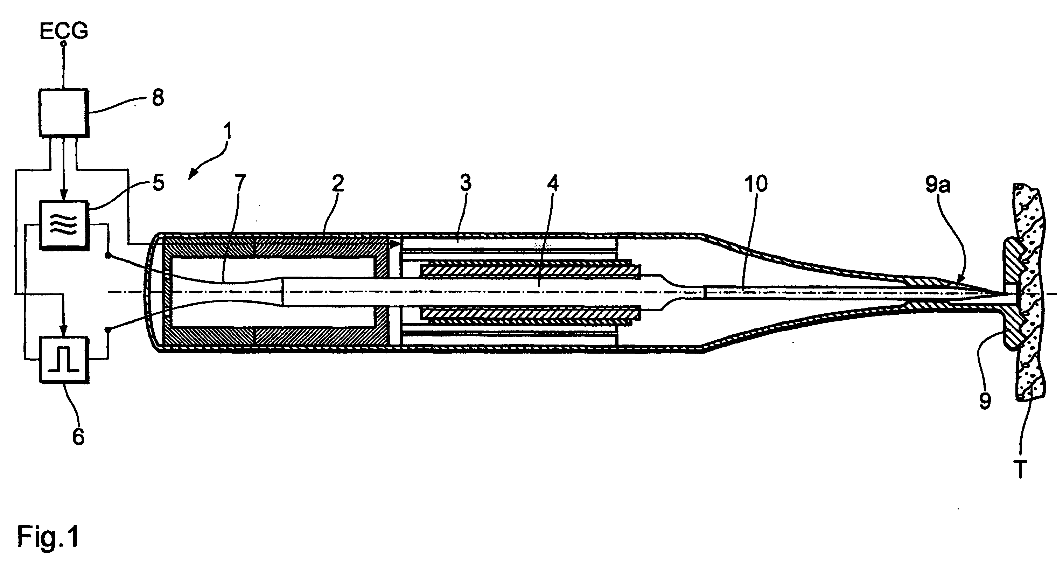

Fig. 1 zeigt als Beispiel für eine erfindungsgemäße Vorrichtung ein Applikationssystem 1 mit einem Handstück bzw. Applikator (im weiteren Sinne) 2, einer darin aufgenommenen elektrischen Antriebseinheit 3 und einer HF-Baugruppe 4, die mittels der Antriebseinheit 3 linear in axialer Richtung auf einen Körpergewebsbereich T hin verschiebbar ist.

Distal angekoppelt an die HF-Baugruppe 4 ist die bipolare Punktionsnadel 10 (der Applikator im engeren Sinne), die sowohl die von einem steuerbaren HF-Generator 5 als auch die von einem steuerbaren Hochspannungs-Impulsgenerator (Funkenstoßwellengenerator) 6 erzeugte elektrische Energie an ein umgebendes Gewebe (nicht gezeigt) abgeben kann. Der elektrische Anschluß der HF-Baugruppe 4 mit der Punktionsnadel 10 an die Spannungserzeuger 5, 6 erfolgt über flexible Zuleitungen 7. Zur Ansteuerung des Antriebes 3 sowie der Spannungserzeuger 5, 6 in Abhängigkeit von die Herzbewegungen reflektierenden EKG-Signalen ECG ist eine Ablaufsteuereinheit 8 vorgesehen.

Ein am distalen Ende des Handstücks 2 vorgesehener Positionierteller 9 mit Gewebefixiereinrichtung ermöglicht über einen seitlich offenen Bereich 9a die präzise visuell unterstützte Positionierung des Applikators 10 im Operationsfeld.

Für eine Revaskularisierungs-Behandlung des Herzens wird das Handstück 2 mit dem Positionierteller 9 an einen ausgewählten Epikard-Bereich angesetzt und - nach Vorwahl der Behandlungsparameter, wie HF-Leistung, Impulsamplitude und Impuls-Repetitionsfrequenz sowie Vorschubbeschwindigkeit etc. - in Synchronisierung zur Herztätigkeit mittels der Ablaufsteuerung 8 und des Antriebes 3 die Punktionsnadel 10 in das Gewebe gestoßen und zugleich HF-Leistung sowie ein Spannungspuls in dieses eingetragen und anschließend die Punktionsnadel wieder zurückgezogen.

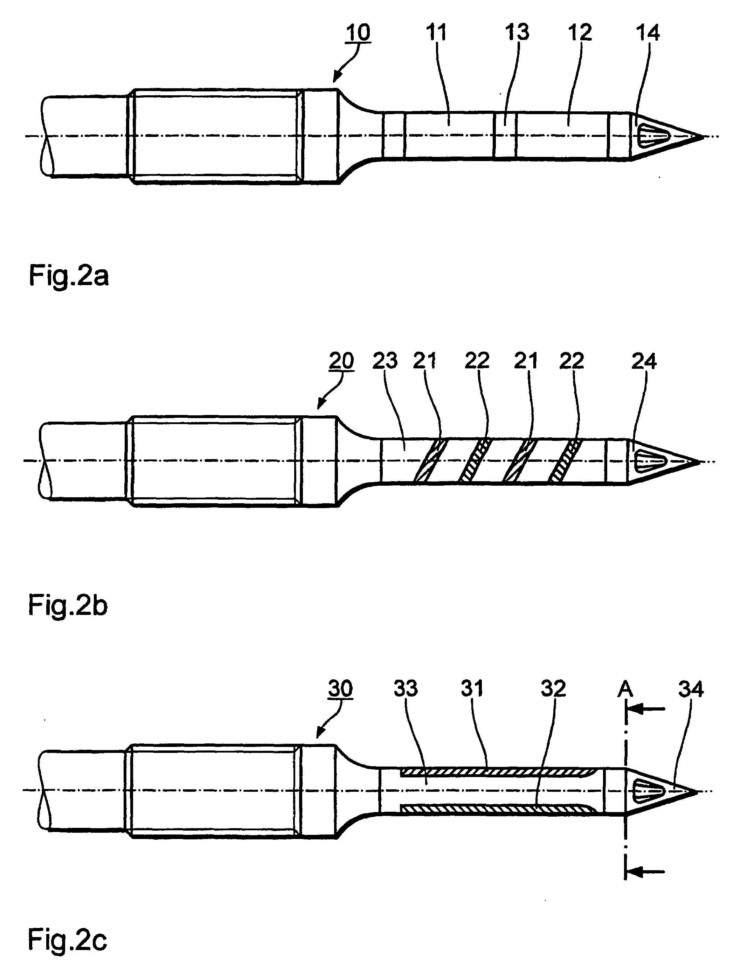

Fig. 2a bis 2c zeigen verschiedene Ausführungsformen von HF-Applikatoren (Punktionsnadeln) 10, 20 bzw. 30, die in einem Gesamtaufbau eingesetzt werden, der grundsätzlich dem in Fig. 1 gezeigten und oben beschriebenen Aufbau entspricht.

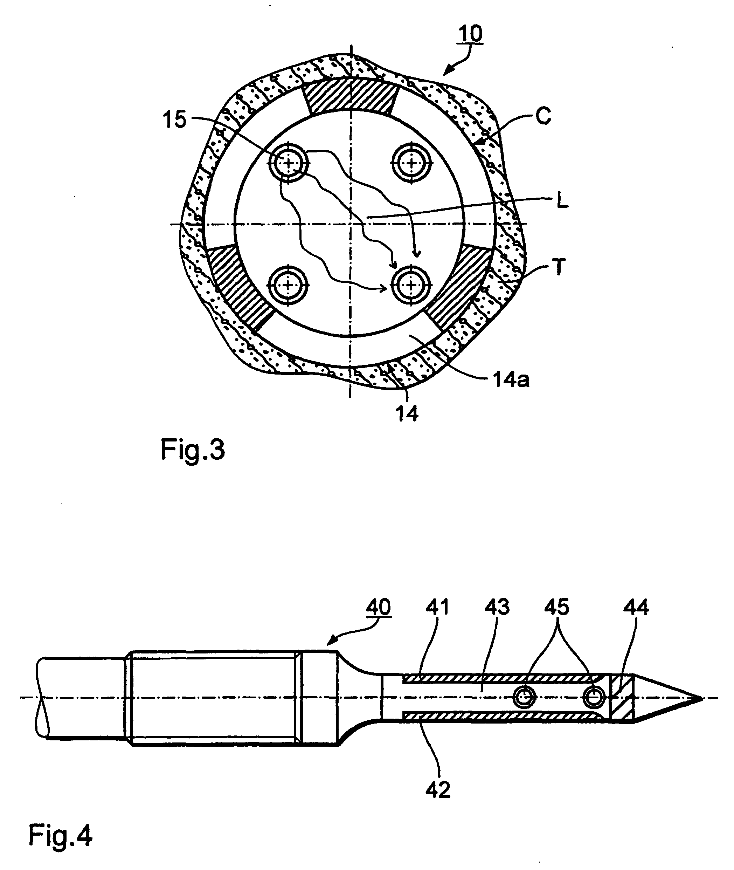

Der Applikator 10 in Abb. 2a weist zwei in Längsachsenrichtung hintereinander angeordnete Elektroden 11, 12 mit gleicher Länge und gleichem Durchmesser, getrennt durch einen Isolatorbereich 13, auf. Am distalen Ende befindet sich eine Hohlspitze 14 mit seitlichen Öffnungen 14a. Innerhalb dieser Spitze sind Impulselektroden angeordnet (siehe Fig. 3).

Der Applikator 20 gemäß Fig. 2b besitzt zwei bifilar auf den Grundkörper 23 gewickelte Elektroden 21, 22, die im Einsatz auf unterschiedliches Potential gelegt werden. Eine Hohlspitze 24 am distalen Ende hat den gleichen Aufbau wie oben zu Fig. 2a beschrieben.

Der Applikator 30 in Fig. 2c besitzt zwei auf einem dielektrischen Grundkörper 33 parallel angeordnete, sich in axialer Richtung erstreckende Elektroden 31, 33. Auch dieser Applikator 30 hat am distalen Ende eine Hohlspitze 34, wie oben beschrieben.

In Fig. 3 ist ein Querschnitt des Übergangsbereiches zwischen dem zylindrischen Abschnitt und der Hohlspitze der HF-Applikatoren längs der Schnittebene A-A dargestellt.

Dort sind vier Impulselektroden 15 derart beabstandet angeordnet, daß sich zwischen ihnen pulsartig eine Lichtbogenentladung L ausbilden kann, deren Energie durch die Öffnungen 14a der Spitze 14 ins umgebende Gewebe T, in dem mittels des Applikators ein Kanal C gebildet wurde, gelangt und eine Stoßwelle erzeugt. Die Enden der Impulselektroden 15 liegen im Bereich der Schnittebene.

In Fig. 4 ist als weitere Ausführungsform ein Applikator 40 dargestellt, bei dem die HF-Elektroden 41, 42 auf dem Trägerkörper 43 wie bei der Ausführung nach Fig. 2c angeordnet sind, bei dem Impulselektroden 45 aber nicht in einer Hohlspitze, sondern galvanisch getrennt auf der Applikatormantelfläche in deren zylindrischem Bereich angeordnet sind. Die Spitze 44 ist hier massiv aus biokompatiblem Kunststoff gefertigt.

Die Erfindung beschränkt sich in ihrer Ausführung nicht auf die vorstehend angegebenen bevorzugten Ausführungsbeispiele. Vielmehr ist eine Anzahl von Varianten möglich, welche von der dargestellten Lösung auch in anders gearteten Ausführungen Gebrauch machen.

So weist eine vereinfachte Ausführung eine manuelle Ablaufsteuerung des Punktionsnadelvorschubes und des Anlegens der HF- und Impulsspannung über einen Taster am Handstück auf. Am ruhenden Herzen können auch mit dieser Ausführung befriedigende Ergebnisse erzielt werden.

## Patentansprüche

1. Vorrichtung (1) zur Revaskularisation von Muskelgewebe (T), insbesondere Herzmuskelgewebe,
**gekennzeichnet durch**
Mittel (6 bis 8; 11, 12, 15; 41, 42, 45) zur voneinander getrennten Realisierung und Steuerung eines Wärmeeintrags zur Erzielung eines thermischen Effektes, insbesondere einer thermischen Randnekrose in einem im Muskelgewebe erzeugten Kanal (C), und einer Stoßwellenwirkung in dem Muskelgewebe.

2. Vorrichtung nach Anspruch 1, **dadurch gekennzeichnet, daß** steuerbare Mittel (5; 11, 12; 21, 22; 31, 32; 41, 42) zur bipolaren Applikation von Hochfrequenzenergie zum Bewirken des Wärmeeintrags und steuerbare Mittel (6; 15; 45) zur Erzeugung und Übertragung elektrischer Impulse an das Muskelgewebe zur Stoßwellenerzeugung vorgesehen sind.

3. Vorrichtung nach Anspruch 2, **dadurch gekennzeichnet, daß** die Mittel (6; 15; 45) zur Erzeugung und Übertragung elektrischer Impulse einen elektrischen Funkenstoßwellengenerator (6) umfassen.

4. Vorrichtung nach einem der vorhergehenden Ansprüche, **gekennzeichnet durch** einen Applikator (10; 20; 30), an dessen Oberfläche mindestens zwei mit einer HF-Quelle (6) verbindbare erste Elektroden (11, 12; 21, 22; 31, 32) vorgesehen sind und der eine Hohlspitze (14; 24; 34) aufweist, in der mindestens zwei mit einer Impulsspannungsquelle (7) verbindbare zweite Elektroden (15) angeordnet sind.

5. Vorrichtung nach einem der Ansprüche 1 bis 3, **gekennzeichnet durch** einen Applikator (40), an dessen Oberfläche mindestens zwei mit einer HF-Quelle (5) verbindbare erste Elektroden (41, 42) und mindestens zwei mit einer Impulsspannungsquelle (6) verbindbare zweite Elektroden (45) angeordnet sind.

6. Vorrichtung nach Anspruch 4 oder 5, **dadurch gekennzeichnet, daß** die steuerbaren Mittel (5) zur bipolaren Applikation von Hochfrequenzenergie und/oder die steuerbaren Mittel (6; 15; 45) zur Erzeugung und Übertragung elektrischer Impulse an das Muskelgewebe eine Einrichtung (3, 8) zur Positionsänderung der ersten und/oder zweiten Elektroden aufweisen.

7. Vorrichtung nach Anspruch 6, **dadurch gekennzeichnet, daß** die Einrichtung (3, 8) zur Positionsänderung der ersten und/oder zweiten Elektroden eine Antriebseinrichtung zur Axialverschiebung entlang der Applikatorlängsachse (A) aufweist.

8. Vorrichtung nach Anspruch 6 oder 7, **dadurch gekennzeichnet, daß** die Einrichtung (3, 8) zur Positionsänderung eine manuelle oder automatische, insbesondere durch Herzsignale (ECG) getriggerte Steuereinrichtung (8) aufweist.

## Claims

1. A device (1) for the revascularisation of muscular tissue (T), in particular myocardial tissue,
**characterised by** means (6 to 8; 11, 12, 15; 41, 42, 45) for
the mutually separated accomplishment and control of the input of heat to achieve a thermal effect, in particular a thermal marginal necrosis in a channel (C) produced in the muscular tissue, and a shock wave effect in the muscular tissue.

2. A device according to Claim 1,
**characterised in that** controllable means (5; 11, 12; 21, 22; 31, 32; 41, 42) are provided for the bipolar application of high-frequency energy to effect the input of heat and controllable means (6; 15; 45) are provided for the production and transmission of electric pulses to the muscular tissue for the production of shockwaves.

3. A device according to one of the preceding Claims,
**characterised in that** the means (6; 15; 45) for generating and transmitting electric pulses comprise an electrical spark discharge shock wave generator.

4. A device according to one of the preceding Claims,
**characterised by** an applicator (10; 20; 30), on the surface of which at least two first electrodes (11, 12; 21, 22; 31, 32) connectable with an HF source (6) are provided and which comprises a hollow point (14; 24; 34), in which at least two second electrodes (15) connectable with a pulse voltage source (7) are disposed.

5. A device according to Claim 4 or 5,
**characterised by** an applicator (40), on the surface of which at least two first electrodes (41, 42) connectable with a HF source (5) and at least two second electrodes (45) connectable with a pulse voltage source (6) are disposed.

6. A device according to Claim 4 or 5,
**characterised in that** the controllable means (5) for the bipolar application of high-frequency energy and/or the controllable means (6; 15; 45) for generating and transmitting electrical pulses to the muscular tissue comprise a device (3, 8) for changing the position of the first and/or second electrodes.

7. A device according to Claim 6,
**characterised in that** the device (3, 8) for changing the position of the first and/or second electrodes comprises a drive mechanism for axial displacement along the longitudinal axis of the applicator (A).

8. A device according to Claim 6 or 7,
**characterised in that** the device (3, 8) for changing the position comprises a manual or an automatic control mechanism (8), in particular triggered by cardiac signals (ECG).

## Revendications

1. Dispositif de revascularisation de tissus musculaires (T), notamment de tissus du muscle cardiaque, **caractérisé par**
des moyens (6 à 8; 11, 12, 15; 41, 42, 45) pour réaliser et commander, séparément les uns des autres, un apport de chaleur pour obtenir un effet thermique, notamment une nécrose thermique des bords dans un canal (C) formé dans le tissu musculaire, et une action d'onde de choc dans le tissu musculaire.

2. Dispositif selon la revendication 1, **caractérisé en ce que** des moyens commandables (5; 11, 12; 21, 22; 31, 32; 41, 42) pour l'application bipolaire d'une énergie à haute fréquence pour réaliser l'introduction de chaleur et des moyens commandables (6; 15; 45) pour produire et transmettre des impulsions électriques au tissu musculaire sont prévus pour la production d'ondes de choc.

3. Dispositif selon la revendication 2, **caractérisé en ce que** les moyens (6; 15; 45) pour produire et transmettre des impulsions électriques comprennent un générateur électrique d'ondes de choc à étincelles (6).

4. Dispositif selon l'une des revendications précédentes, **caractérisé par** l'applicateur (10; 20; 30), sur une surface duquel sont prévues au moins deux premières électrodes (11, 12; 21, 22; 31, 32), qui peuvent être reliées à une source à haute fréquence (6) et qui comportent une pointe creuse (14; 24; 34), dans laquelle sont disposées au moins deux secondes électrodes (15) pouvant être reliées à une source de tension impulsionnelle (7).

5. Dispositif selon l'une des revendications 1 à 3, **caractérisé par** un applicateur (40), sur la surface duquel sont disposées au moins deux premières électrodes (41, 42) pouvant être reliées à une source à haute fréquence (5), et au moins deux secondes électrodes (45) pouvant être reliées à une source de tension impulsionnelle (6) .

6. Dispositif selon la revendication 4 ou 5, **caractérisé en ce que** les moyens commandables (5) pour l'application bipolaire d'une énergie à haute fréquence et/ou les moyens commandables (6; 15; 45) pour la production et la transmission d'impulsions électriques au tissu musculaire comportent un dispositif (3, 8) pour modifier la position des premières et/ou secondes électrodes.

7. Dispositif selon la revendication 6, **caractérisé en ce que** le dispositif (3, 8) servant à modifier la position de la première et/ou de la seconde électrode comporte un dispositif d'entraînement pour réaliser un déplacement axial le long de l'axe longitudinal (A) de l'applicateur.

8. Dispositif selon la revendication 6 ou 7, **caractérisé en ce que** le dispositif (3, 8) comporte, pour le changement de position, un dispositif de commande manuel ou automatique (8) déclenché notamment par des signaux cardiaques (ECG).
